# EUROPÄISCHE PATENTSCHRIFT

(11) **EP 1 334 983 B1**
(45) Veröffentlichungstag und Bekanntmachung des Hinweises auf die Patenterteilung: **12.04.2006**
(21) Anmeldenummer: 03000857.7
(22) Anmeldetag: 15.01.2003
(51) Int. Cl.: C07K 16/36, C12N 5/20, C07K 2/00, C07K 14/745, A61K 39/395, G01N 33/577, A61P 7/02

(54) **Inhibitorischer, monoklonaler Antikörper gegen die den Blutgerinnungsfaktor VII akitivierende Protease**
Inhibitory, monoclonal antibody against the protease which activates clotting factor VII
Anticorps monoclonal inhibiteur, dirigé contre la protéase activant le facteur de coagulation VII

(30) Priorität: 08.02.2002 DE 10205520
(43) Veröffentlichungstag der Anmeldung: 13.08.2003
(73) Patentinhaber: ZLB Behring GmbH, 35041 Marburg (DE)
(72) Erfinder: Römisch, Jürgen, Dr., 2331 Vösendorf (AT); Lang, Wiegand, 35091 Coelbe (DE); Feussner, Annette, 35043 Marburg (DE); Muth-Naumann, Gudrun, 35083 Wetter (DE); Stoehr, Hans-Arnold, 35083 Wetter (DE); Kannemaier, Christian, 35390 Giessen (DE); Preissner, Klaus, 35394 Giessen (DE); Nakazawa, Fumie, Tokyo 112-0002 (JP)

(56) Entgegenhaltungen:
- EP-A- 1 059 359
- EP-A- 1 182 258
- DE-A- 19 903 693
- ROEMISCH J ET AL: "QUANTIATION OF THE FACTOR VII- AND SINGLE-CHAIN PLASMINOGEN ACTIVATOR-ACTIVATING PROTEASE IN PLASMAS OF HEALTHY SUBJECTS" BLOOD COAGULATION & FIBRINOLYSIS, RAPID COMMUNICATIONS, OXFORD,OXFORD, GB, Bd. 12, Nr. 5, Juli 2001 (2001-07), Seiten 375-383, XP009013812 ISSN: 0957-5235

## Beschreibung

Gegenstand der Erfindung ist ein monoklonaler Antikörper, der spezifisch die den Faktor VII aktivierende Protease oder ihr Proenzym inhibiert, die proteolytischen Eigenschaften anderer Proteasen jedoch nicht beeinträchtigt.

Es ist bekannt, dass Proteasen im Organismus eine entscheidende Funktion bei der Freisetzung aktiver Enzyme aus ihren Vorstufen, den Proenzymen, haben. Darüber hinaus haben Proteasen aber auch die Eigenschaft, aktivierte Enzyme wieder abzubauen, so dass diese häufig schon nach kurzer Zeit ihre Wirkung wieder verlieren. Zur Herstellung stabilisierter pharmazeutischer Zubereitungen ist es deshalb häufig erforderlich, die Aktivität spezieller Proteasen zu inhibieren.

Die Stabilisierung gegen den durch Proteasen hervorgerufenen Abbau ist deshalb auch bei pharmazeutischen Präparaten, die Blutgerinnungsfaktoren enthalten, eine wichtige Aufgabe, um die Wirksamkeit derartiger Präparate insbesondere auch noch nach einer längeren Lagerung sicherzustellen.

Das Blutgerinnungssystem umfasst zwei unterschiedliche, kaskadenförmige Aktivierungswege von im Plasma anwesenden Gerinnungsfaktoren. Je nach auslösendem Mechanismus dient bevorzugt der endogene oder der exogene Weg zur Initiation der Gerinnung.

Bei einer Gewebeverletzung wird als Initiator des exogenen Gerinnungsweges das Thromboplastin (tissue factor, TF) an Oberflächen exponiert. An das membranständige Thromboplastin wird der Gerinnungsfaktor VII (FVII) gebunden, ebenso wie zirkulierender, aktivierter FVII (FVIIa). Dieser TF-FVIIa-Komplex führt in Gegenwart von Kalziumionen und Lipiden zur Bindung des FX, der durch limitierte Proteolyse in seine aktivierte Form (FXa) überführt wird. FXa führt wiederum durch Aktivierung von Prothrombin zu Thrombin zur Bildung von Fibrin und damit letztendlich zum Wundverschluss.

FVIIa wird in sehr geringen Konzentrationen in Plasma gesunder Menschen gefunden. Über die Ursache und Herkunft des in Blut zirkulierenden FVIIa ist bisher nur wenig bekannt. Spuren von exprimiertem oder bei einer Zellzerstörung freigesetztem Thromboplastin könnten dabei eine Rolle spielen.

Aus der deutschen Offenlegungsschrift 199 03 693 ist eine FVII aktivierende Protease bekannt geworden, die auch als FSAP (Faktor Sieben Aktivierende Protease) bezeichnet wird. Aufgrund dieser Eigenschaft kann FSAP die Blutgerinnung beschleunigen und als Pharmazeutikum bei Blutungskomplikationen Anwendung finden.

FSAP kommt in Blutplasma als Proenzym (single-chain FSAP, scFSAP) vor. Durch bekannte chromatographische Verfahren kann man hauptsächlich nur die zweikettige aktivierte Form der Protease (two-chain FSAP, tcFSAP) gewinnen, da das Proenzym während der Präparation aktiviert wird. Diese Aktivierung kann durch andere Proteasen wie die Urokinase erfolgen, tritt aber auch autokatalytisch ein. Die aktivierte FSAP kann sich selbst proteolytisch inaktivieren. Deshalb ist insbesondere die Herstellung der scFSAP schwierig, aber auch die Herstellung der intakten tcFSAP ist nicht einfach. Optimierte Verfahren zur Herstellung von FSAP sind in den deutschen Offenlegungsschriften 139 37 218 und 139 37 219 beschrieben worden. Ein wichtiger Verfahrensschritt ist dabei die Immunadsorption an matrixgekoppelte, monoklonale Antikörper und spezielle Bedingungen zur Stabilisierung des Eluates. Aber auch bei der Immunadsorption ist darauf zu achten, dass möglichst rasch gearbeitet wird, um selbst das Entstehen geringer tcFSAP-Mengen zu vermeiden. Das gelingt am Besten, wenn bei der Präparation anstelle von polyvalenten Proteinasehemmem wie Aprotinin, C1-Esterase-Inhibitor oder α-2-Antiplasmin spezifische monoklonale Antikörper eingesetzt werden, die nur die Aktivierung oder Eigenaktivierung von FSAP verhindern oder vermindern.

Gegenstand der Erfindung ist deshalb ein monoklonaler Antikörper gegen FSAP der die den Blutgerinnungsfaktor VII aktivierende Protease oder ihr Proenzym inhibiert. Hierfür eignet sich insbesondere der monoklonale Antikörper, der von der Hybridoma-Zelllinie DSM ACC 2533 gebildet wird. Wird ein derartiger monoklonaler Antikörper der den Blutgerinnungsfaktor VII aktivierenden Protease oder ihrem Proenzym zugesetzt, dann werden diese hierdurch inhibiert. Eine Faktor VII-Zubereitung, die den vorstehend genannten monoklonalen Antikörper enthält, wird durch die Inhibition der den Faktor VII aktivierenden Protease und ihres Proenzyms stabilisiert.

Der erfindungsgemäße Antikörper kann auch für präparative und analytische Anwendungen eingesetzt werden. Es ist nämlich aus der deutschen Offenlegungsschrift 199 03 693 bekannt, das FSAP die Eigenschaft hat, bei einer Inkubation die Blutgerinnungsfaktoren VIII/VIIIa und V/Va in einer von der Proteasekonzentration und der Inkubationsdauer abhängigen Weise zu inaktivieren. Hemmt man nun die Wirkung von FSAP durch Zusatz des erfindungsgemäßen Antikörpers, dann werden dadurch die genannten Blutgerinnungsfaktoren gegen den proteolytischen Abbau geschützt. Auch Fibrinogen Lösungen kann man durch Zusatz des erfindungsgemäßen Antikörpers stabilisieren.

Diagnostische Einsatzmöglichkeiten werden durch den erfindungsgemäßen inhibitorischen Antikörper dadurch eröffnet, dass man bei seiner Zugabe zu einer Proteasen enthaltenden Lösung durch die selektive Hemmung des FSAP erkennen kann, ob die proteolytische Wirkung auf FSAP oder eine andere Protease zurückgeführt werden kann. Dieses Verfahren kann im Besonderen bei allen Testsystemen angewendet werden, die auf der Aktivität von FSAP beruhen, zum Beispiel der Spaltung von chromogenen Substraten, durch aktivierte FSAP oder durch Messung der amidolytischen Aktivität der durch FSAP aktivierten Urokinase.

Darüber hinaus ergeben sich für die erfindungsgemäßen, inhibitorischen Antikörper auch direkte prophylaktische oder therapeutische Einsatzmöglichkeiten bei bestimmten Erkrankungen. So kann beispielsweise eine Erhöhung des FSAP-Gehaltes im Plasma die prokoagulatorischen Eigenschaften des Blutes und damit das Thromboserisiko erhöhen. Durch Verabreichung eines inhibitorischen Antikörpers kann eine Verminderung der Gerinnungsfähigkeit des Blutes und damit eine Verminderung des Thromboserisikos erreicht werden.

In der deutschen Offenlegungsschrift 19 903 693 wird auch die besondere fibrinolytische Wirkung von pharmazeutischen Zubereitungen erwähnt, die die den Blutgerinnungsfaktor VII aktivierende Protease enthalten. Die Protease kann deshalb auch zur Behandlung von Erkrankungen eingesetzt werden, die durch fibrinhaltige Thromben verursacht werden. Hieraus geht hervor, dass FSAP in höheren Konzentrationen eine Neigung zu Blutungen auslösen oder verstärken kann, die durch einen inhibitorischen, monoklonalen Antikörper verhindert oder vermindert werden kann. Damit können auch negative Einflüsse auf die Wundheilung oder die Entstehung von Krebs inhibiert werden.

Die vielfältigen beschriebenen Anwendungsmöglichkeiten eines inhibitorischen monoklonalen Antikörpers gegen FSAP werden dadurch erreicht, dass der Antikörper die Aktivierung von scFSAP verhindert und sich dann das tcFSAP nicht mehr bilden kann. Da viele der bisher bekannten Eigenschaften von FSAP durch das tcFSAP vermittelt werden, reicht die Inhibierung des scFSAP aus, um die Wirkungen des FSAP zu blockieren.

Der efindungsgemäß aus der Hybridomazelle DSM ACC 2533 gewonnene monoklonale Antikörper hemmt deshalb sehr effektiv die Aktivität der tcFSAP, aber auch die Autoaktivierung der scFSAP. Es ist selbstverständlich, dass dieser monoklonale Antikörper bei einer Anwendung als Prophylaktikum oder Therapeutikum beim Menschen vorher noch humanisiert werden muss. Diese Verfahren sind allgemein bekannt und sollen hier nicht im einzelnen beschriebenen werden.

In der deutschen Patentanmeldung 100 52 319.6 ist außerdem bereits beschrieben, dass neben der den Blutgerinnungsfaktor VII aktivierenden Protease (FSAP) bei 5 bis 10% aller untersuchten Blutspender hinsichtlich der scuPA-Aktivierung eine inaktive Mutante von FSAP vorkommt, deren Aminosäuresequenz an der Aminosäureposition 393 einen Glu/Gln-Austausch und Gly/Glu-Austausch aufweist. Mit diesem "single-nucleotide polymorphism" (=SNP) ist eine Reduktion der Aktiverung von Pro-Plasminogenaktivatoren und damit ein vermindertes fibrinolytisches Potential sowie ein erhöhtes Thromboserisiko verbunden. Um dieses Risiko zu vermindern, wird in der europäischen Patentanmeldung 01 115 691.6 die Verabreichung der Wildtyp FSAP vorgeschlagen.

Die vorliegende Erfindung bietet nun die weitere Möglichkeit, durch Verabreichung eines inhibitorischen, monoklonalen Antikörpers, die Inhibition von FSAP und damit auch die der Mutante zu bewirken, wodurch das thrombotische bzw. thromboembolische Potential reduziert wird. Andererseits können Mutanten des FSAP, deren FVII aktivierende Eigenschaften vermindert und dafür deren fibrinolytische Kapazität gesteigert sind, zur Behandlung von potentiell erhöhten Blutungsneigungen durch die Verabreichung eines erfindungsgemäßen monoklonalen, inhibitorischen Antikörpers eingesetzt werden.

Die Hybridomazelllinie DSM ACC 2533 wurde auf folgende Weise identifiziert und präpariert: 3 Mäuse wurden mit FSAP immunisiert. Die Milzzellen einer Maus wurden mit der murinen Myelom-Zelllinie SP2/0-Ag 14 mit Polyethylenglykol 4.000 als Fusionsreagenz fusioniert. Die Zellen wurden auf 24 Well-Kulturplatten verteilt. Als Medium wurde Dulbecco mod. Eagle's Medium mit 10% fötalem Kälberserum und HAT zur Selektion verwendet. Nach etwa 2 Wochen wurden die wachsenden Zellklone in die Vertiefungen einer 48 Well-Kulturplatte transferiert und kodiert. Von 1.728 gewachsenen Zellklonen wurde der Überstand mittels ELISA auf Anwesenheit von Maus-lgG getestet. Mit Hilfe von immobilisierter FSAP wurde Maus-lgG positive Überstände auf Spezifität geprüft. Von den getesteten Klonen wurde 108 als spezifisch für FSAP identifiziert. In weiteren Experimenten wurde die inhibitorische Eigenschaft des monoklonalen Antikörpers aus der Hybridoma-Zelllinie DSM ACC 2533 gefunden. Der Antikörper ist vom IgG1-Typ.

Der monoklonale Antikörper aus der Hybridoma-Zelllinie DSM ACC 2533 inhibiert die proteolytische Aktivität von tcFSAP. Entsprechend hemmt er nicht nur die Fähigkeit von FSAP, den Faktor VII oder Prourokinase zu aktivieren, sondern verhindert ebenfalls die Eigenaktivierung von FSAP.

Die Erfindung wird durch die folgenden Beispiele näher erläutert:

### Beispiel 1

### Untersuchung der Einflüsse des monoklonalen Antikörpers aus DSM ACC 2533 auf die (Eigen)Aktivierung von scFSAP

Die Rekalzifizierung von Zitrat-Plasma führt zur Aktivierung der Gerinnung und schließlich zur Formation eines Fibrin-Gerinnsels. Unter diesen Umständen wird scFSAP nicht oder nur stark verzögert aktiviert. Durch Simulation von sog. Kontaktaktivatoren oder reaktiven Oberflächen durch Zugabe von Dextransulfat wird scFSAP auch im Plasmamilieu aktiviert. Dies lässt sich anhand von SDS-PAGE und Wester-Blotting-Analysen sehr anschaulich darstellen. Dazu wird die rekalzifizierte und mit Dextransulfat versetzte Probe für unterschiedliche Zeiten inkubiert und anschließend einer sog., dem Fachmann vertrauten, Euglobulin-Fällung unterzogen, um die anschließende SDS-PAGE optimal ausführen zu können. Auf diese Weise wird die Proteinkonzentration der Proben gesenkt und das Verschmieren von Bandenmustern verhindert. Die so erhaltenen Proben werden mit einem Reduktionsmittel versetzt, um einfacher die durch Aktivierung von scFSAP entstehenden schweren und leichten Ketten der tcFSAP zu identifizieren. Nach Transfer der durch SDS-PAGE erhaltenen Bandenmuster auf Nitrocellulose (Blotting) werden die in der deutschen Patentanmeldung 100 36 641.4 beschriebenen monoklonalen Antikörper DSM ACC 2453 und DSM ACC 2454 in markierter Form zur Detektion der beiden tcFSAP Banden verwendet.

Als Resultat dieser Versuchsausführung erkennt man das Verschwinden der scFSAP auf dem Blot und die Zunahme der beiden tcFSAP-Ketten. Gelegentlich kann auch nur die schwere Kette sichtbar werden, da die leichte Kette der tcFSAP das aktive Zentrum der FSAP enthält und während der Inkubationszeit des Plasmas mit entsprechenden Inhibitorproteinen, wie C1-Esterase-Inhibitor oder α-2-Antiplasmin, reagiert und schließlich als Komplex (quasi-kovalent) schlechter von dem detektierenden, monoklonalen Antikörper erkannt wird.

Versetzt man die oben beschriebene Plasmaprobe mit dem aus DSM ACC 2533 gewonnenen monoklonalen Antikörper und verfährt man zur Detektion der scFSAP-Aktivierung wie oben beschrieben, so erkennt man im Gegensatz zur Kontrolle (ohne den genannten monoklonalen Antikörper oder mit einem nichtinhibitorischen, monoklonalen Antikörper) kein Verschwinden der hochmole- kularen (Einkettenform) Bande und entsprechend nicht das Auftreten der schweren (und leichten) Kette(n) der tcFSAP. Der monoklonale Antikörper aus der Hybridoma-Zelllinie der DSM ACC 2533 hat also an scFSAP gebunden und verhindert so die Aktivierung der scFSAP.

Die Titration dieses Effektes zeigt, dass der genannte Antikörper ein hochpotenter, monoklonaler Antikörper zur Inhibition der scFSAP-Aktivierung ist. Bereits in equimolaren Mengen zu FSAP findet eine signifikante Reduktion der Aktivierung statt und macht diesen inhibitorischen, monoklonalen Antikörper zu einem sehr wertvollen diagnostischen und präparativen Hilfsmittel. Die hohe Effektivität bei relativ geringer Konzentration macht eine potentielle Verwendung dieses monoklonalen Antikörpers zur Humanapplikation attraktiv.

### Beispiel 2

### Inhibition der aktiven tcFSAP durch den inhibitorischen, monoklonalen Antikörper aus der Hybridoma-Zelllinie DSM ACC 2533

Wie in der deutschen Patentanmeldung 199 03 693 beschrieben, kann die Aktivität von FSAP in verschiedenen Assaysystemen getestet werden. Neben der Aktivierung von Plasminogenaktivator-Proenzymen oder des FVII mit entsprechender Beschleunigung der Gerinnung kann beispielsweise auch die Inaktivierungsrate von FV, FVIII, FIX oder Fibrinogen verfolgt werden. In diesen Testsystemen wurde der erfindungsgemäße inhibitorische, monoklonale Antikörper auf seine inhibitorischen Eigenschafen geprüft.

Als Ergebnis ist festzuhalten, dass der inhibitorische, monoklonale Antikörper aus der Hybridoma-Zelllinie DSM ACC 2533 in potenter Weise die Aktivität von tcFSAP gegenüber den vorstehend genannten Blutgerinnungsfaktoren hemmt. Dies gilt auch für scFSAP, dessen Aktivierung zu tcFSAP und damit seine Konversion in die amidolytisch aktive Form verhindert wird, wie es im vorstehenden Beispiel 1 gezeigt wird.

## Patentansprüche

1. Monoklonaler Antikörper gegen FSAP, **dadurch gekennzeichnet, dass** er die den Blutgerinnungsfaktor VII aktivierende Protease (FSAP) und ihr Proenzym durch spezifische Bindung an FSAP und ihr Proenzym inhibiert.

2. Monoklonaler Antikörper nach Anspruch 1, **dadurch gekennzeichnet, dass** er von der Hybridoma-Zelllinie DSM ACC 2533 gebildet wird.

3. Hybridoma-Zelllinie, die einen Antikörper gemäß Anspruch 1 produziert.

4. Hybridoma-Zellinie nach Anspruch 3, **dadurch gekennzeichnet, dass** es die unter der Zugangsnummer DSM ACC 2533 hinterlegte Hybridoma-Zelllinie ist.

5. Monoklonaler Antikörper nach Anspruch 1, **dadurch gekennzeichnet, dass** seine Bindung an FSAP durch den monoklonalen Antikörper gemäß Anspruch 2 inhibiert wird.

6. Single-chain Antikörper, **dadurch gekennzeichnet, dass** er die Bindungsspezifität des Antikörpers nach Anspruch 2 hat

7. Antikörper nach Anspruch 6, **dadurch gekennzeichnet, dass** er an ein Trägerprotein, bevorzugterweise Humanserumalbumin oder humanes Transferrin gebunden ist

8. Humanisierter Antikörper, **dadurch gekennzeichnet, dass** er die Bindungsspezifität des Antikörpers nach Anspruch 2 hat.

9. FSAP und/oder FSAP-Proenzym-Zubereitung, **dadurch gekennzeichnet, dass** sie einen monoklonalen Antikörper nach den Ansprüchen 1 oder 2 enthält, der die den Faktor VII aktivierende Protease und ihr Proenzym inhibiert

10. Faktor VII-Zubereitung, **dadurch gekennzeichnet, dass** sie einen monoklonalen Antikörper nach den Ansprüchen 1 oder 2 enthält, der die den Faktor VII aktivierende Protease und ihr Proenzym inhibiert

11. Stabilisierte Faktor V-, Faktor VIII- oder Fibrinogen-Zubereitung, **dadurch gekennzeichnet, dass** sie gegen den proteolytischen Abbau durch die den Faktor VII aktivierende Protease durch die Inhibition dieser Protease mittels eines monoklonalen Antikörpers der Ansprüche 1 oder 2 geschützt ist.

12. Pharmazeutische Zubereitung, **dadurch gekennzeichnet dass** sie einen monoklonalen Antikörper der Ansprüche 1 oder 2 enthält

13. Pharmazeutische Zubereitung gemäß Anspruch 12 zur Verminderung der Gerinnungsfähigkeit des Blutes, **dadurch gekennzeichnet, dass** sie einen monoklonalen Antikörper der Ansprüche 1 oder 2 enthält

14. Verfahren zur Bestimmung der Aktivität der den Blutgerinnungsfaktor VII aktivierenden Protease, **dadurch gekennzeichnet, dass** man die in der zu untersuchenden Probe enthaltene Menge an Proteasen durch Zusatz einer ausreichenden Menge eines monoklonalen Antikörpers der Ansprüche 1 oder 2 stabilisiert und erst dann die verbliebene amidolytische Aktivität misst.

15. Verfahren zur Präparation von FSAP oder des FSAP Proenzyms, **dadurch gekennzeichnet, dass** der monoklonale Antikörper gemäß Ansprüchen 1 und 2 als Immunadsorbens verwendet wird.

16. Verfahren zur Präparation von FSAP oder des FSAP Proenzyms, **dadurch gekennzeichnet, dass** der monoklonale Antikörper gemäß Ansprüchen 1 und 2 dem Ausgangsmaterial zugesetzt wird, aus dem FSAP gewonnen wird.

## Claims

1. A monoclonal antibody against FSAP which inhibits blood clotting factor VII-activating protease (FSAP) and its proenzyme by specific binding to FSAP and its proenzyme.

2. The monoclonal antibody as claimed in claim 1, which is produced by hybridoma cell line DSM ACC 2533.

3. A hybridoma cell line, which produces an antibody as claimed in claim 1.

4. The hybridoma cell line as claimed in claim 3, which is the hybridoma cell line deposited under accession number DSM ACC 2533.

5. The monoclonal antibody as claimed in claim 1, whose binding to FSAP is inhibited by the monoclonal antibody as claimed in claim 2.

6. A single-chain antibody, which has the binding specificity of the antibody as claimed in claim 2.

7. The antibody as claimed in claim 6, which is bound to a carrier protein, preferably human serum albumin or human transferin.

8. A humanized antibody, which has the binding specificity of the antibody as claimed in claim 2.

9. An FSAP and/or FSAP proenzyme preparation, which contains a monoclonal antibody as claimed in either of claims 1 and 2, which antibody inhibits the factor VII-activating protease and its proenzyme.

10. A factor VII preparation, which contains a monoclonal antibody as claimed in either of claims 1 and 2, which antibody inhibits the factor VII-activating protease and its proenzyme.

11. A stabilized factor V, factor VIII or fibrinogen preparation, which is protected against proteolytic degradation by factor VII-activating protease due to inhibition of said protease by means of a monoclonal antibody of either of claims 1 and 2.

12. A pharmaceutical preparation, which contains a monoclonal antibody of either of claims 1 and 2.

13. The pharmaceutical preparation as claimed in claim 12 for reducing the coagulability of blood, which contains a monoclonal antibody of either of claims 1 and 2.

14. A method for determining the activity of blood clotting factor VII-activating protease, wherein the amount of proteases contained in the sample to be studied is stabilized by the addition of a sufficient amount of a monoclonal antibody of either of claims 1 and 2 and the amidolytic activity remaining is measured only thereafter.

15. A method for preparing FSAP or the FSAP proenzyme, which uses the monoclonal antibody as claimed in either of claims 1 and 2 as immunoadsorbent.

16. A method for preparing FSAP or the FSAP proenzyme, wherein the monoclonal antibody as claimed in either of claims 1 and 2 is added to the starting material from which FSAP is obtained.

## Revendications

1. Anticorps monoclonal dirigé contre la FSAP, **caractérisé en ce qu'**il inhibe la protéase activant le facteur de coagulation VII (FSAP) et sa proenzyme par liaison spécifique à une FSAP et à sa proenzyme.

2. Anticorps monoclonal selon la revendication 1, **caractérisé en ce qu'**il est formé par la lignée cellulaire d'hybridome DSM ACC 2533.

3. Lignée cellulaire d'hybridome qui produit un anticorps selon la revendication 1.

4. Lignée cellulaire d'hybridome selon la revendication 3, **caractérisée en ce que** c'est la lignée cellulaire d'hybridome déposée sous le numéro de dépôt DSM ACC 2533.

5. Anticorps monoclonal selon la revendication 1, **caractérisé en ce que** sa liaison à FSAP est inhibée par l'anticorps monoclonal selon la revendication 2.

6. Anticorps monocaténaire, **caractérisé en ce qu'**il a la spécificité de liaison de l'anticorps selon la revendication 2.

7. Anticorps selon la revendication 6, **caractérisé en ce qu'**il est lié à une protéine support, de préférence une albumine de sérum humaine ou la transferrine humaine.

8. Anticorps humanisé, **caractérisé en ce qu'**il a la spécificité de liaison de l'anticorps selon la revendication 2.

9. FSAP et/ou composition de FSAP-proenzyme, **caractérisée en ce qu'**elle comprend un anticorps monoclonal selon les revendications 1 ou 2, qui inhibe la protéase activant le facteur VII et sa proenzyme.

10. Composition de facteur VII, **caractérisée en ce qu'**elle comprend un anticorps monoclonal selon les revendications 1 ou 2, qui inhibe la protéase activant le facteur VII et sa proenzyme.

11. Composition de facteur V, facteur VIII ou de fibrinogène stabilisée, **caractérisée en ce qu'**elle est protégée de la dégradation protéolytique opérée par la protéase activant le facteur VII grâce à l'inhibition de cette protéase au moyen d'un anticorps monoclonal selon les revendications 1 ou 2.

12. Composition pharmaceutique, **caractérisée en ce qu'**elle comprend un anticorps monoclonal selon les revendications 1 ou 2.

13. Composition pharmaceutique selon la revendication 12, pour réduire la faculté de coagulation du sang, **caractérisée en ce qu'**elle contient un anticorps monoclonal selon les revendications 1 ou 2.

14. Procédé pour la détermination de l'activité de la protéase activant le facteur de coagulation du sang VII, **caractérisé en ce que** l'on stabilise la quantité de protéases contenue dans l'échantillon à analyser par addition d'une quantité suffisante d'un anticorps monoclonal selon les revendications 1 ou 2 et que l'on mesure ensuite seulement l'activité amidolytique résiduelle.

15. Procédé de préparation de FSAP ou de proenzyme FSAP, **caractérisé en ce que** l'anticorps monoclonal selon les revendications 1 et 2 est utilisé comme immunoadsorbant.

16. Procédé de préparation de FSAP ou de proenzyme FSAP, **caractérisé en ce que** l'anticorps monoclonal selon les revendications 1 et 2 est additionné à la matière de départ, dont est tirée la FSAP.
